(19) Europäisches Patentamt
European Patent Office
Office européen des brevets

(11) Veröffentlichungsnummer: **0 354 246 B1**

(12) **EUROPÄISCHE PATENTSCHRIFT**

(45) Veröffentlichungstag der Patentschrift: **16.03.94**

(51) Int. Cl.[5]: **C07H 19/073**, C07H 19/10, C07H 19/173, C07H 19/20, A61K 31/70

(21) Anmeldenummer: **89901341.1**

(22) Anmeldetag: **20.12.88**

(86) Internationale Anmeldenummer: **PCT/SU88/00271**

(87) Internationale Veröffentlichungsnummer: **WO 89/06238 (13.07.89 89/15)**

# (54) 3'-Azido-2',3'-dideoxynucleosid-5'-phosphonate.

(30) Priorität: **29.12.87 SU 4404761**

(43) Veröffentlichungstag der Anmeldung: **14.02.90 Patentblatt 90/07**

(45) Bekanntmachung des Hinweises auf die Patenterteilung: **16.03.94 Patentblatt 94/11**

(84) Benannte Vertragsstaaten: **CH DE FR GB LI SE**

(56) Entgegenhaltungen:
**EP-A- 199 451**
**EP-A- 217 580**
**DE-A- 3 608 606**
**US-A- 4 681 933**

(73) Patentinhaber: **INSTITUT MOLEKULYARNOI BIOLOGII IMENI V.A. ENGELGARDTA AKADEMII NAUK SSSR**
**ul. Vavilova 32**
**Moscow, 117984(SU)**

Patentinhaber: **INSTITUT VIRUSOLOGII IMENI D.I.IVANOVSKOGO AKADEMII NAUK SSSR**
**ul. Gamalei 16**
**Moscow, 123098(US)**

Patentinhaber: **VSESOJUZNY KARDIOLOGICHESKY NAUCHNY TSENTR AKADEMII MEDITSINSKIKH NAUK SSSR**
**ul. 3 Cherepkovskaya, 15a**
**Moscou, 121552(SU)**

(72) Erfinder: **KHORLIN, Alexandr Anatolievich**
**ul. Generala Antonova, 7-1-131**
**Moscow, 117342(SU)**
Erfinder: **TARUSOVA, Natalya Borisovna**
**ul. Zhivopisnaya, 50-40**
**Moscow, 123098(SU)**
Erfinder: **DYATKINA, Natalya Borisovna**
**ul. Generala Antonova, 7-1-131**
**Moscow, 117342(SU)**
Erfinder: **KRAEVSKY, Alexandr Antonovich**
**ul. Profsojuznaya, 132-4-111**
**Moscow, 117321(SU)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **BIBILASHVILI, Robert Shalvovich**
**pr. Kutuzovsky, 43-85**
**Moscow, 121170(SU)**
Erfinder: **GALEGOV, Georgy Artemievich**
**ul. Smolnaya, 31-46**
**Moscow, 125195(SU)**
Erfinder: **KORNEEVA, Marina Nikolaevna**
**ul. Sakko i Vantsetti, 30-83**
**Moskovskaya obl. Kaliningrad, 141070(SU)**
Erfinder: **NOSIK, Dmitry Nikolaevich**
**ul. Miklukho-Maklaya, 53-99**
**Moscow, 117279(SU)**
Erfinder: **MAIOROVA, Svetlana Nikolaevna**
**Strelbischensky per., 26/9-5**
**Moscow, 123317(SU)**
Erfinder: **SHOBUKHOV, Vadim Maximovich**
**ul. Alabiana, 10-216**
**Moscow, 125080(SU)**
Erfinder: **ZHDANOV, Viktor Mikhailovich**

**deceased(SU)**

(74) Vertreter: **von Füner, Alexander, Dr. et al**
**Patentanwälte v. Füner, Ebbinghaus, Finck**
**Mariahilfplatz 2 & 3**
**D-81541 München (DE)**

## Beschreibung

Die erfindung bezieht sich auf ein Gebiet der molekularen Biologie und betrifft insbesondere neue Verbindungen-3'-Azido-2',3'-didesoxynukleosid-5'-phosphonate, die selektive Hemmstoffe der Reproduktion des Virus des Immunodefizites beim Menschen in der Kultur der menschlichen Lymphozyten darstellen.

Heutzutage sind verschiedene Verbindungen bekannt, die die Reproduktion von Virus des Immunodefizites beim Menschen hemmen. Von den bekannten Verbindungen ist 3'-Azido-3'-didesoxythymidin (AzT), das in der medizinischen Praxis Verwendung findet, am effektivsten (Mitsuya et al., Proc. Natl. Acad. Sci., USA, 1985, 82, 7096-7100; M.A.Fischl et al., New England J. Medicine, 1987, 317, 185-191; D.D.Richman et al., New England J.Medicine, 1987, 317, 192-197).

Der molekulare Wirkungsmechanismus der angegebenen Verbindung AzT schließt die Diffusion in die durch das Virus des menschlichen Immunodefizites infizierten Zeilen ein. Weiterhin wird sie der Triphosphorylierung unterzogen und blockiert spezifisch die Synthese von DNS, die durch die Rücktranskriptase katalysiert wird, welche mit dem Virus des menschlichen Immunodefizites kodiert wird. AzT hemmt jedoch die Reproduktion von Virus des menschlichen Immunodefizites nicht in allen Zellentypen des Menschen, was offensichtlich auf den verschiedenen Umwandlungsgrad von AzT-5'-triphosphat (AzTTP) zurückzuführen ist. Die genannte Verbindung AzT wird durch die Toxizität gekennzeichnet, die hauptsächlich die Blutbildung und das Zentralnervensystem beeinflußt. Andere 3'-Azido-2',3'-didesoxynukleoside mit Basen: Cytosin (AzC), Adenin (AzA) und Guaninin (AzG) sind auch in bezug auf die Hemmung der Reproduktion von Virus des menschlichen Immunodefizites aktiv, zwar weniger als AzT.

Die vorgeschlagene Verbindungen sind neu und wurden in der Fachliteratur nicht beschrieben.

Der Erfindung liegt die Aufgabe zugrunde, neue Verbindungen zu entwickeln, die eine selektive Aktivität in bezug auf die Hemmung der Reproduktion von Virus des menschlichen Immunodefizites und eine niedrige Toxizität besitzen.

Die Aufgabe wurde dadurch gelöst, daß erfindungsgemäß neue Verbindungen - 3'-Azido-2',3'-didesoxynukleosid-5'-phosphonate der allgemeinen Formel vorgeschlagen werden:

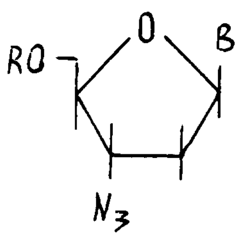

worin bedeuten:

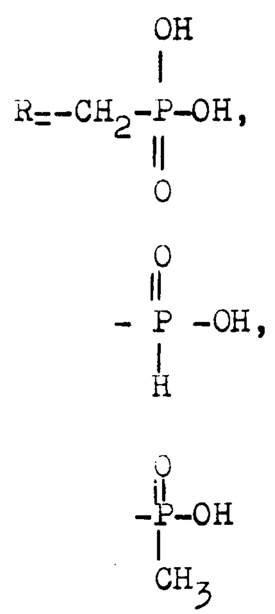

B = Thymin-I-yl, Cytosin-I-yl, Adenina9-yl, Guanin-9-yl.

Die genannten Verbindungen besitzen die Fähigkeit, die Reproduktion von Virus des Immunodefizites beim Menschen zu hemmen und weisen eine im Vergleich zu bekannten Arzneimitteln geringere Toxizität auf.

Die beste Ausführungsform der Erfindung

Die erfindungsgemäßen Verbindungen stellen weiße amorphe Pulver dar, die gut wasserlöslich, im Äthylalkohol begrenzt löslich, in anderen organischen Lösungsmitteln unlöslich sind.

Der Reinheitsgrad und die Struktur der erfindungsgemäßen Verbindungen werden durch die Angaben der Chromatographie, der UV-Spektren und der 'H-NMR-Spektren bestätigt.

Die erfindungsgemäßen Verbindungen legen in vitro die Eigenschaften an den Tag, die Reproduktion von Virus des Immunodefizites beim Menschen in der Kultur der menschlichen Lymphozyten N9/ShV und MT4 zu hemmen.

Es wurden die Eigenschaften der erfindungsgemäßen Verbindungen untersucht, die Reproduktion von Virus des Immunodefizites beim Menschen zu hemmen.

Als Quelle des Virus des menschlichen Immunodefizites wurden transplantierbare menschliche lymphoblastoide Linien der Zellen der Produzenten des erwahnten Virus (N9/ShV und MT4) ausgenutzt. Die Virusproduktion durch die Zellen wurde in der Reaktion der Immunofluoreszenz und nach der elektronenmikroskopischen Methode geprüft. Die Zellen wurden im Medium RPMI 1640 mit dem 15%igen inaktivierten Embryonenserum der Kühe, mit 300 µg/ml Glutamin, 100 µg/ml Gentamyzin, 10 mMol HEPES-Puffer kultiviert und in Form von Suspension gezüchtet. Die Kulturflüssigkeit wurde durch Zentrfugieren der Zellen bei 5000 U/min innerhalb von 10 Minuten gesammelt und zur Infizierung der Lymphozyten des peripheren Blutes wurden gesunde Blutspender in einer Menge von I ml Supernatant der Kulturflüssigkeit ausgenutzt, die däs Virus des menschlichen Immunodefizites pro jede $10^6$ Lymphozyten des peripheren Blutes des Menschen enthielt, die im Gradienten Fikoll-Isopan aus dem heparinisierten Blut der gesunden Blutspender isoliert wurden. Die Zellen wurden im Medium RPMI 1640 mit dem 15%igen inaktivierten Embryonenserum der Kühe, 300 µg/ml Glutamin, 100 µg/ml Gentamyzin, 100 mMol HEPES-Puffer kultiviert und innerhalb von 48 Stunden bei einer Temperatur von 37 °C in der befeuchteten $CO_2$-Atmosphäre in Gegenwart von Phytohämagglutinin in einer Konzentration von 100 µg/ml inkubiert. Die nachfolgende Kultivierung der Lymphozyten nach dem Eintragen des Virus wurde innerhalb von 6 Tagen in Gegenwart des 10%igen menschlichen natürlichen lymphozytären Interleukin-2 durchgeführt.

Die Bestimmung der antigenen Aktivität (Immunofermentanalyse) des Materials wurde in Kunststoffpaneelen mit 96 Aushöhlungen in an sich bekannter Weise durchgeführt. Die Reaktion der indirekten Immunofluoreszenz wurde in fixierten Präparaten der Antigen-haltigen Zellen - Produzenten des Virus des menschlichen Immunodefizites durchgeführt. Die Berechnung der lebensfähigen Zellen wurde unter Ausnutzung des Farbstoffes Trypanblau verwirklicht.

Die Untersuchungsergebnisse sind in Tabellen 1 bis 4 angeführt.

Als Kontrollen wurden Azidonukleoside AzT, AzC, AzA bzw. AzG verwendet.

Tabelle 1.

Vergleichende Untersuchung der Aktivität, der erfindungsgemäßen Verbindungen und des 3'-Azido-2',3'-didesoxythymidins (AzT) in der Hemmung der Reproduktion des Virus des menschlichen Immunodefizites in den Zellen N9/ShV.

| Lfd. Nr. | Bedingungen des Versuchs | Erfindungsgemäße Verbindungen: AzT (Kontrolle): Konz. der Zellen in 1 ml (x 10⁶) | AzT (Kontrolle): Virushaltige Zellen: Zellen/ml | AzT (Kontrolle): Virushaltige Zellen: % | 3'-Azido-2',3'-didesoxythymidin-5'-methylenphosphonat: Konz. der Zellen in 1 ml (x10⁶) | 3'-Azido-2',3'-didesoxythymidin-5'-methylenphosphonat: Virushaltige Zellen: Zellen/ml | 3'-Azido-2',3'-didesoxythymidin-5'-methylenphosphonat: Virushaltige Zellen: % |
|---|---|---|---|---|---|---|---|
| 1 | 2 | 3 | 4 | 5 | 6 | 7 | 8 |
| 1. | Kontrolle | 1,20 | - | - | 1,1 | - | - |
| 2. | Kontrolle+Virus | 0,60 | 0,28 | 46 | 1,0 | 0,52 | 52 |
| 3. | Kontrolle+Virus+1 µMol Prüfsubstanz | 0,60 | 0,06 | 10 | 0,75 | 0,11 | 15 |
| 4. | Kontrolle+Virus+5 µMol Prüfsubstanz | 0,58 | 0,06 | 11 | | | |
| 5. | Kontrolle+Virus+ 10 µMol Prüfsubstanz | 0,35 | 0,035 | 10 | 1,0 | 0,06 | 6 |

Fortsetzung der Tabelle 1

| Lfd. Nr. | Erfindungsgemäße Verbindungen: 3'-Azido-2',3'-didesoxythymidin-5'-hydrophosphonat: Konz. der Zellen in 1 ml (x10⁶) | 3'-Azido-2',3'-didesoxythymidin-5'-hydrophosphonat: Virushaltige Zellen: Zellen/ml | 3'-Azido-2',3'-didesoxythymidin-5'-hydrophosphonat: Virushaltige Zellen: % | 3'-Azido-2',3'-didesoxythymidin-5'-methylphosphonat: Konz. der Zellen in 1 ml (x10⁶) | 3'-Azido-2',3'-didesoxythymidin-5'-methylphosphonat: Virushaltige Zellen: Zellen/ml | 3'-Azido-2',3'-didesoxythymidin-5'-methylphosphonat: Virushaltige Zellen: % |
|---|---|---|---|---|---|---|
| 1 | 9 | 10 | 11 | 12 | 13 | 14 |
| 1. | 1,20 | - | - | 1,1 | - | - |
| 2. | 0,60 | 0,21 | 36 | 1,0 | 0,52 | 52 |
| 3. | 0,65 | 0,12 | 18 | 1,2 | 0,26 | 22 |
| 4. | 0,60 | 0,13 | 22 | | | |
| 5. | 1,0 | 0,14 | 14 | 0,75 | 0,13 | 17 |

Tabelle 2.

Vergleichende Untersuchung der Aktivität der erfindungsgemäßen Verbindung und des 3'-Azido-2',3'-didesoxycytidins (AzC) in der Hemmung der Reproduktion von Virus des menschlichen Immunodefizites in den Zelle MT4

| Lfd. Nr. | Bedingungen des Versuches | AzC (Kontrolle): Konz. der Zellen in 1 ml (x$10^6$) | AzC (Kontrolle): Virushaltige Zellen, Zellen/ml | AzC (Kontrolle): Virushaltige Zellen, % | Erfindungsgemäße Verbindung 3'-Azido-2',3'-didesoxycytidin-5'-hydrophosphonat: Konz. der Zellen in 1 ml (x$10^6$) | Erfindungsgemäße Verbindung: Virushaltige Zellen, Zellen/ml | Erfindungsgemäße Verbindung: Virushaltige Zellen, % |
|---|---|---|---|---|---|---|---|
| 1 | 2 | 3 | 4 | 5 | 6 | 7 | 8 |
| 1. | Kontrolle | 1,03 | - | - | 1,03 | - | - |
| 2. | Kontrolle+Virus | 0,42 | 0,42 | 100 | 0,42 | 0,42 | 100 |
| 3. | Kontrolle+Virus+10 µMol Prüfsubstanz | 0,46 | 0,45 | 97 | 0,52 | 0,48 | 92 |
| 4. | Kontrolle+Virus+30 µMol Prüfsubstanz | 0,40 | 0,36 | 91 | 0,73 | 0,57 | 78 |

Tabelle 3.

Vergleichende Untersuchung der Aktivität der erfindungsgemäßen Verbindungen und des 3'-Azido-2',3'-didesoxyadenosins (AzA) in der Hemmung der Reproduktion von Virus des menschlichen Immunodefizites in den Zellen MT4

| Lfd. Nr. | Bedingungen des Versuchs | AzA (Kontrolle) Konz. der Zellen in 1 ml (x10⁶) | AzA (Kontrolle) Virushaltige Zellen: Zellen/ml | AzA (Kontrolle) Virushaltige Zellen: % | Erfindungsgemäße Verbindungen: 3'-Azido-2',3'-didesoxyadenosin-5'-methylenphosphonat: Konz. der Zellen in 1 ml (x10⁶) | Virushaltige Zellen: Zellen/ml | Virushaltige Zellen: % |
|---|---|---|---|---|---|---|---|
| 1 | 2 | 3 | 4 | 5 | 6 | 7 | 8 |
| 1. | Kontrolle | 0,95 | - | - | 0,95 | - | - |
| 2. | Kontrolle+Virus | 0,48 | 0,46 | 96 | 0,48 | 0,48 | 96 |
| 3. | Kontrolle+Virus+5 µMol Prüfsubstanz | 0,45 | 0,42 | 92 | 0,48 | 0,29 | 61 |
| 4. | Kontrolle+Virus+10 µMol Prüfsubstanz | 0,52 | 0,45 | 86 | 0,65 | 0,35 | 56 |
| 5. | Kontrolle+Virus+30 µMol Prüfsubstanz | 0,56 | 0,45 | 80 | 0,94 | 0,30 | 32 |

Fortsetzung der Tabelle 3

| Lfd. Nr. | Erfindungsgemäße Verbindungen: 3'-Azido-2',3'-didesoxyadenosin-5'-hydrophosphonat: Konz. der Zellen in 1 ml (x10⁶) | Virushaltige Zellen: Zellen/ml | Virushaltige Zellen: % | 3'-Azido-2',3'-didesoxyadenosin-5'-methylphosphonat: Konz. der Zellen in 1 ml (x10⁶) | Virushaltige Zellen: Zellen/ml | Virushaltige Zellen: % |
|---|---|---|---|---|---|---|
| 1 | 9 | 10 | 11 | 12 | 13 | 14 |
| 1. | 0,95 | - | - | 0,95 | - | - |
| 2. | 0,48 | 0,46 | 96 | 0,48 | 0,46 | 96 |
| 3. | 0,56 | 0,36 | 65 | 0,51 | 0,45 | 88 |
| 4. | 0,62 | 0,40 | 65 | 0,58 | 0,47 | 81 |
| 5. | 0,92 | 0,35 | 38 | 0,61 | 0,41 | 67 |

Tabelle 4.

Vergleichende Untersuchung der Aktivität der erfindungsgemäßenen Verbindungen und des 3'-Azido-2',3'-didesoxyguanosins (AzG) in der Hemmung der Reproduktion von Virus des menschlichen Immunodefizites in den Zellen MT4

| Lfd. Nr. | Bedingungen des Versuchs | AzG (Kontrolle) | | | Erfindungsgemäße Verbindungen 3'-Azido-2',3'-didesoxyguanosin-5'-methylenphosphonat | | |
|---|---|---|---|---|---|---|---|
| | | Konz. der Zellen in 1 ml ($x10^6$) | Virushaltige Zellen Zellen/ml | % | Konz. der Zellen in 1 ml ($x10^6$) | Virushaltige Zellen Zellen/ml | % |
| 1 | 2 | 3 | 4 | 5 | 6 | 7 | 8 |
| 1. | Kontrolle | 1,2 | - | - | 1,2 | - | - |
| 2. | Kontrolle+Virus | 0,65 | 0,65 | 100 | 0,65 | 0,65 | 100 |
| 3. | Kontrolle+Virus+1 µMol Prüfsubstanz | 0,65 | 0,63 | 96 | 0,68 | 0,62 | 91 |
| 4. | Kontrolle+Virus+5 µMol Prüfsubstanz | 0,71 | 0,68 | 96 | 0,70 | 0,48 | 68 |
| 5. | Kontrolle+Virus+10 µMol Prüfsubstanz | 0,76 | 0,73 | 96 | 0,85 | 0,26 | 30 |
| 6. | Kontrolle+Virus+30 µMol Prüfsubstanz | 0,81 | 0,73 | 90 | 1,04 | 0,13 | 12 |

Fortsetzung der Tabelle 4

| Lfd. Nr. | Erfindungsgemäße Verbindungen: 3'-Azido-2',3'-didesoxyguanosin-5'hydrophosphonat | | | 3'-Azido-2',3'-didesoxyguanosin-5'-methylphosphonat | | |
|---|---|---|---|---|---|---|
| | Konz. der Zellen in 1 ml ($x10^6$) | Virushaltige Zellen Zellen/ml | % | Konz. der Zellen in 1 ml ($x10^6$) | Virushaltige Zellen Zellen/ml | % |
| 1 | 9 | 10 | 11 | 12 | 13 | 14 |
| 1. | 1,20 | - | - | 1,20 | - | - |
| 2. | 0,65 | 0,65 | 100 | 0,65 | 0,65 | 100 |
| 3. | 0,62 | 0,60 | 96 | 0,65 | 0,65 | 100 |
| 4. | 0,75 | 0,21 | 28 | 0,70 | 0,43 | 61 |
| 5. | 0,86 | 0,20 | 23 | 0,78 | 0,45 | 58 |
| 6. | 1,07 | 0,11 | 10 | 0,56 | 0,38 | 68 |

Wie aus den in Tabellen gegebenen Ergebnissen zu ersehen ist, blockieren die erfindungsgemaßen Verbindungen 3'-Azido-2',3'-didesoxynukleosid-5'-methylenphosphonate oder -5'-hydrophosphonate in allen Fällen effektiv die Reproduktion von Virus des menschlichen Immunodefizites, indem sie dabei die Vermehrung der Zellen bedeutend schwächer als entsprechende Azidonukleoside hemmen. Indem die erfindungsgemäßen Verbindungen 3'-Azido-2',3'-didesoxynukleosid-5'-methylphosphonate selbst eine antivirale Wirkung ausüben, rufen sie eine bedeutende Hemmung des Zellenwachstums infolge der Toxizität hervor, was sich in der Verminderung der Zellenpopulation im Vergleich zu 3'-Azido-2',3'-didesoxynukleosid-5'-methylenphosphonaten oder-5'-hydrophosphonaten äußert.

Dadurch weisen alle erfindungsgemäßen Verbindungen einen ausgeprägten antiviralen Effekt in bezug auf das Virus des menschlichen Immunodefizites auf. Es wurde die Toxizität der erfindungsgemäßen

Verbindungen untersucht. Die Ergebnisse sind in der Tabelle 5 angeführt, woraus zu ersehen ist, daß die erfindungsgemäßen Verbindungen eine niedrigere Toxizität im Vergleich zu den Ausgangsazidonukleosiden (AzT, AzC, AzA, AzG) besitzen.

Tabelle 5.

Toxizität der erfindungsgemäßen Verbindungen im Vergleich zu den Azidonukleosiden in den Kulturen der menschlichen Leukozyten N9/ShV und MT4

| Lfd. Nr. | Die Verbindung | Wachstumshemmung bei einer Konzentration von 0,5 mMol,% | |
|---|---|---|---|
| | | N9/ShV | MT4 |
| 1 | 2 | 3 | 4 |
| 1. | 3'-Azido-2',3'-didesoxythymidin 5'-methylenphosphonat | 35 | 10 |
| 2. | 3'-Azido-2',3'-didesoxycytidin-5'-methylenphosphonat | 20 | 65 |
| 3. | 3'-Azido-2',3'-didesoxyadenosin-5'-methylenphosphonat | 10 | 30 |
| 4. | 3'-Azido-2',3'-didesoxyguanosin-5'-methylenphosphonat | 30 | 65 |
| 5. | 3'-Azido-2',3'-didesoxythymidin-5'-hydrophosphonat | 10 | 30 |

Fortsetzung der Tabelle 5

| 1 | 2 | 3 | 4 |
|---|---|---|---|
| 6. | 3'-Azido-2',3'-didesoxycythidin 5'-hydrophosphonat | 10 | 10 |
| 7. | 3'-Azido-2',3'-didesoxyadenosin-5'-hydrophosphonat | 10 | 0 |
| 8. | 3'-Azido-2',3'-didesoxyguanosin-5'-hydrophosphonat | 20 | 30 |
| 9. | 3'-Azido-2',3'-didesoxythymidin-5'-methylphosphonat | 85 | 100 |
| 10. | 3'-Azido-2',3'-didesoxycytidin-5'-methylphosphonat | 85 | 100 |
| 11. | 3'-Azido-2',3'-didesoxyadenosin-5'-methylphosphonat | 70 | 80 |
| 12. | 3'-Azido-2',3'-didesoxyguanosin-5'-methylphosphonat | 80 | 100 |
| 13. | AzT | 100 | 100 |
| 14. | AzC | 80 | 60 |
| 15. | AzA | 70 | 80 |
| 16. | AzG | 90 | 100 |

Die erfindungsgemäßen Verbindungen werden folgenderweise hergestellt.

3'-Azido-2',3'-didesoxynukleosid-5'-methylenphosphonate werden durch Umsetzung des Oxymethylphosphonsäure-P,P-diäthylester-toluolsulfonats mit dem Azidonukleosid oder mit einem N-geschützten Azidonukleosidderivat erhalten, die mit Natriumhydrid im Medium eines organischen Lösungsmittels in der Atmosphäre eines inerten Gases behandelt werden. Nachher wird das Endprodukt aus der Reaktionsmasse isoliert und gereinigt. Die Ausbeute an Endprodukt beträgt 25 bis 40 Ma%.

3'-Azido-2',3'-didesoxynukleosid-5'-hydrophosphonate werden durch Umsetzung von Imidazol, Phosphortichlorid und Azidonukleosid oder einem N-geschützten Azidonukleosidderivat im organischen Lösungsmittel und darauffolgende Isolierung und Reinigung des Endproduktes erhalten. Die Ausbeute an Endprodukt beträgt 58 bis 80 Ma%.

3'-Azido-2',3'-didesoxynukleosid-5'-methylphosphonate werden durch Umsetzung von Dichlormethylphosphonat mit Azidonukleosid oder einem N-geschützten Azidonukleosidderivat im Medium eines organischen Lösungsmittels und darauffolgende Isolierung und Reinigung des Endproduktes erhalten. Die Ausbeute an Endprodukt beträgt 30 bis 40 Ma%.

Zum besseren Verständnis der vorliegenden Erfindung werden folgende Beispiele der Herstellung der erfindungsgemäßen Verbindungen angeführt.

Beispiel 1.

Zu einer Suspension von Natriumhydrid (54 mg, 2,25 mMol) in 3 ml Dimethylformamid gibt man innerhalb von 15 bis 20 Minuten in der Atmosphäre eines inerten Gases die Lösung von 3'-Azido-2',3'-didesoxythymidin (200 mg, 0,75 mMol) in 5 ml Dimethylformamid zu, rührt innerhalb von 30 Minuten bei einer Temperatur von 20 °C. Dann gibt man die Lösung von Oxymethylphosphonsäure-P,P-diäthylester-toluolsulfonat (240 mg, 0,75 mMol) in 3 ml Dimethylformamid hinzu und rührt innig die Reaktionsmasse innerhalb von 3 Tagen bei einer Temperatur von 20 °C. Den Reaktionsverlauf kontrolliert man nach der dünnschichtschromatografischen Methode im System Chloroform-Alkohol in einem Verhältnis von 9:1 (das System A). Nach der Beendigung der Reaktion setzt man 0,13 ml Essigsäure zu, dampft die Reaktionsmasse ein, dampft nochmals mit 5 ml Dimethylformamid ein. Den Rückstand extrahiert man mit Chloroform (5x5 ml). Man dampft Extrakte ein, reinigt den Rückstand auf einer Säule mit Silikagel 40/100 $\mu$ (12x2,5 cm). Man führt die Elution mit 300 ml Chloroform, dann mit 400 ml Chloroform-Äthanol-Gemisch (9:1) durch, indem die Fraktionen zu 2 ml gesammelt werden. Man sammelt entsprechende Fraktionen und dampft ein. Man erhält 130 mg Verbindung in Form von Öl mit $R_f$ von0,63 (System A), die man in 3 ml Dimethylformamid löst; man gibt Trimethylsilybromid (0,3 ml) bei einer Temperatur von 4 °C hinzu, rührt das Gemisch innerhalb von 30 Minuten und läßt noch für 24 Stunden bei einer Temperatur von 20 °C stehen. Man dampft die Reaktionsmasse ein, gibt 5 ml Wasser und Triäthylamin bis zum Erhalten eines pH-Wertes von 9,0 zu, läßt für 1 Stunde stehen, extrahiert mit Chloroform (3x5 ml). Man dampft die wäßrige Schicht ein, reinigt den Rückstand auf einer Säule mit dem Zellstoff DEAE (in der $NCO_3^-$ -Form) von 300 ml Inhalt im linearen Gradienten von Ammoniumhydrogenkarbonat von 0 bis 0,3 M, das Gesamtvolumen des Eluenten beträgt 3 l. Man dampft die das Endprodukt enthaltenden Fraktionen ein, dampft diese mit Wasser (5x10ml) und Äthanol (3x20 ml)ein. Man extrahiert den Rückstand mit 10 ml Methanol, dampft bis zum Erhalten eines Volumens von 2 ml ein, gibt 30 mg $NaClO_4$ und 5 ml Azeton hinzu. Man trennt den Niederschlag ab, wäscht mit Azeton, trocknet. Man erhält 82 mg (40 Ma%) 3'-Azido-2', 3'-didesoxythymidin-5'-methylenphosponat, das eine weiße amorphe Substanz darstellt, die gut wasserlöslich, in organischen Lösungsmitteln unlöslich ist.$R_f$ = 0,2 im System Isopropanol-Ammoniak-Wasser 7:1:2 (das System B). (Hocheffektive Flüssigkeitschromatographie am Nukleosil 120-7 $NH_2$ im linearen Gradienten von $K_2HPO_4$ 0,5- 1 M), die Haltezeit beträgt 10,6 min. Das UV-Spektrum, nm, $\lambda_{max}$265 ($\xi$9600 pHl), $\lambda_{min}$238 ($\xi$3500 pHl); 'H-NMR-Spektrum ($D_2O$, $\delta$ppm); 7,54 d (IH,H6, J = 0,5 Hz); 1,95 d (3H,$CH_3$, J = 0,5 Hz); 6,18 t (IH,HI'$J_{I,2}$, = 6 0Hz); 2,60 m (2H,2H2'); 3,70-4,80 m (4H,H3' + H4' + 2H5');3,68 d (2H,$CH_2P$, $J_{P,H}$ = 8,6 Hz). $^{31}$P-NMR ($D_2O$, $\delta$ppm): 15,7 t ($J_{P,H}$ = 8,6 Hz).

Beispiel 2

Zu einer Suspension von Natriumhydrid (54 mg, 2,25 mMol) in 3 ml Dimethylformamid gibt man innerhalb von 15 bis 20 Minuten in der Atmosphäre eines inerten Gases die Lösung von 3'-Azido-2',3'-didesoxy-N-benzoylcytidin (265 mg, 0,75 mMol) in 5 ml Dimethylformamid hinzu, rührt innerhalb von 30 Minuten bei einer Temperatur von 20 °C. Dann gibt man die Lösung von Oxymethylphosphonsäure-P,P-diäthylester-toluolsulfonat (240 mg,0,75 mMol) in 3 ml Dimethylformamid hinzu und rührt innig die Reaktionsmasse innerhalb von 3 Tagen bei einer Temperatur von 20 °C. Den Reaktionsverlauf kontrolliert man nach der dünnschichtchromatografischen Methode im System A. Nach der Beendigung der Reaktion setzt man 0,13 ml Essigsäure zu, dampft die Reaktionsmasse ein. Man extrahiert den Rückstand mit Chloroform (5x5 ml), dampf Chloroform ein. Man löst den Rückstand in 3 ml Dimethylformamid, gibt 0,3 ml Trimethylsilylbromid bei einer Temperatur von 4 °C hinzu, läßt die Lösung für 24 Stunden bei einer Temperatur von 20 °C stehen. Man dampft die Reaktionsmasse ein, gibt 5 ml Wasser und Triäthylamin bis zum Erzielen eines pH-Wertes von 9,0 hinzu, extrahiert nach 1 Stunde mit Chloroform. Man konzentriert die wäßrige Schicht bis zum Erhalten eines Volumens von 1 ml und setzt 30 ml gesättigte Ammoniaklösung in Methanol zu, um die N-Benzoylgruppe zu entfernen. Nach 24 Stunden dampft man die Lösung ein.

Die Isolierung und Reinigung des Endproduktes führt man ähnlich mit dem Beispiel 1 durch. Man erhält 3'-Azido-2',3'-didesoxycytidin-5'-methylenphosphonat. Die Ausbeute beträgt 45 Ma%.

Die erhaltene Verbindung stellt eine weiße amorphe Substanz dar, die gut wasserlöslich, in organischen Lösungsmitteln unlöslich ist.

$R_f$ = 0,14 (das System B); die Haltezeit beträgt 8,8 min, das UV-Spektrum, nm, pH 7,0: $\lambda_{max}$ 271 ($\xi$8900); $\lambda_{min}$252 ($\xi$6300)'H-NMR ($D_2O$, $\delta$,ppm): 7,66 d (IH,H6,$J_{5,6}$ = 8,0 Hz); 5,79 d (IH, H5, $J_{5,6}$ = 8,0 Hz); 5,95 t (IH,HI', $J_{I',2'}$ = 6,0 Hz); 2,66 m (2H,2H2'); 3,81-4,34 m (4H,H3'H4' + 2H5'); 3,75 d (2H, $CH_2P$, $J_{P,H}$ = 8,6 Hz). $^{31}$P-NMR ($D_2O$,$\delta$, ppm): 16,0 t, $J_{P,H}$ = 8,6 Hz.

Beispiel 3.

Ähnlich mit dem Beispiel 2 erhält man 3'-Azido-2',3'-desoxyadenosin-5'-methylenphosphonat. Die Ausbeute beträgt 35 Ma%.

Die erhaltene Verbindung stellt eine weiße amorphe Substanz dar, die gut wasserlöslich, in organischen Lösungsmitteln unlöslich ist. $R_f$ = 0,12 (das System B), die Haltezeit beträgt 12,4 min. Das UV-Spektrum,

nm, pH 7,0: $\lambda_{max}$ 261 ($\xi$15100), $\lambda_{min}$ 231 ($\xi$2750); 'H-NMR ($D_2O$,$\delta$,ppm): 8,23 s (IH,H2): 8,38 s (IH,H8); 6,40 t (IH,H; $J_{1,2}$ = 5,0 Hz); 2,80 m (2H,2H2'); 4,00-4,50 m (4H,H3 + H4 + 2H5); 3,70 d (2H,$CH_2$P, $J_{P,H}$ = 8,6 Hz). $^{31}$P-NMR ($D_2O$,$\delta$,ppm): 15,7 t, $J_{P,H}$ = = 8,6 Hz).

Beispiel 4.

Ahnlich mit dem Beispiel 2 erhält man 3'-Azido-2',3'-didesoxyguanosin-5'-methylenphosphonat. Die Ausbeute beträgt 27 Ma%.

Die erhaltene Verbindung stellt eine weiße amorphe Substanz dar, die gut wasserlöslich, in organischen Lösungsmitteln unlöslich ist.

$R_f$ = 0,08 (das System B), die Haltezeit beträgt 12,3 min, das UV-Spektrum, nm, pH 7,0; $\lambda_{max}$ 253 ($\xi$11500), $\lambda_{min}$ 227 ($\xi$2900). 'H-NMR ($D_2O$,$\delta$,ppm): 8,10 s (IH,H8); 6,35 t (IH,HI', $J_{I'2'}$ = 5,0 Hz); 2,8 m (2H,2H2'); 3,95-4,40 m (4H,H3' + H4' + 2H5'); 3,65 d (2H,$CH_2$P, $J_{P,H}$ = 8,6 Hz). $^{3I}$P-NMR ($D_2O$,$\delta$,ppm) 15,8 t, $J_{P,H}$ = 8,6 Hz).

Beispiel 5.

Man löst Imidazol (0,50 g, 7,36 mMol) in 15 ml absolutem Azetonitril und kühlt auf 0 °C ab. Man gibt unter Rühren Phosphortrichlorid (0,19 ml, 2,16 mMol) hinzu. Man mischt das Reaktionsgemisch innerhalb von 15 Minuten bei einer Temperatur von 0 °C und setzt nachher innerhalb von 30 Minuten tropfenweise 3'-Azido-2',3'-didesoxythymidin (134 mg, 0,5 mMol) in 10 ml Azetonitril auf die Weise hinzu, daß die Temperatur der Reaktionsmasse + 5 °C nicht übersteigt. Man mischt die Reaktionsmasse innerhalb von 2 Stunden bei einer Temperatur von 20 °C durch, indem 3,5 ml Wasser zugegeben werden, und dampft nach 30 Minuten ein. Danach trägt man die Substanz in eine Säule mit Toeperl DEAE (5x25 cm) auf und eluiert mit Ammoniumnydrogenkarbonat im linearen Konzentrationsgradienten von 0 bis 0,3 M, das Gesamtvolumen ist I I. Man sammelt die das Endprodukt enthaltenden Fraktionen, und dampft ein. Den Überschuß an Salz entfernt man durch das mehrmalige Eindampfen mit Wasser. Den Rückstand lyophilisiert man aus dem Wasser. Man erhält 140 mg 3'-Azido-2',3'-didesoxythymidin-5'hydrophosphonat (80 Ma%), das eine weiße amorphe Substanz darstellt, die gut wasserlöslich, im Äthylalkohol begrenzt löslich ist. $R_f$ = 0,55 (das System B), die Haltezeit beträgt 7,2 min, das UV-Spektrum, nm, pHI: $\lambda_{max}$ 265 ($\xi$9800), $\lambda_{min}$ 238 ($\xi$3400). 'H-NMR-Spektrum ($D_2O$,$\delta$,ppm): 7,52 d (IH,H6, J = 0,5 Hz); 1,92 d (3H,$CH_3$, J = 0,5 Hz); 6,52 t (IH,HI', $J_{I',2'}$ = = 6,0 Hz); 2,45 m (2H,2H2'); 3,90-4,42 m (4H,H3 + H4' + 2H5'); 6,64 d (IH,$H^P$, $J_{P,H}$ = 629 Hz). $^{31}$P-NMR ($D_2O$,$\delta$,ppm): 6,6 m ($J_{P,H}$ = 629 Hz, $J_{P,H}$5' = 6,3 Hz, $J_{P,H}$4 = 1,6 Hz) (das Spektrum wurde ohne Hemmung der Spaltung an Protonen aufgenommen).

Beispiel 6.

Man löst Imidazol (0,50 g, 7,36 mMol) in 15 ml absolutem Azetonitril und kühlt auf 0 °C ab. Man gibt unter Rühren Phosphortrichlorid (0,19 ml, 2,16 mMol) und danach Triäthylamin (1,05 ml, 7,54 mMol) hinzu. Man mischt das Reaktionsgemisch innerhalb von 15 Minuten bei einer Temperatur von 0 °C und setzt nachher innerhalb von 30 Minuten tropfenweise 3'-Azido-2',3'-didesoxybenzoylcytidin (182 mg, 0,5 mMol) in 10 ml Azetonitril auf die Weise hinzu, daß die Temperatur der Reaktionsmasse +5 °C nicht übersteigt. Man mischt die Reaktionsmasse innerhalb von 2 Stunden bei einer Temperatur von 20 °C durch, gibt 3,5 ml Wasser hinzu und dampft nach 30 Minuten ein. Die Entfernung der Benzoylgruppe führt man mit der gesättigten methanolischen Ammoniaklösung bei einer Temperatur von 20 °C innerhalb von 24 Stunden durch. Die Isolierung und Reinigung führt man nach der im Beispiel 5 beschriebenen Methodik durch.

Man erhält 3'-Azido-2',3'-didesoxycytidin-5'-hydrophosphonat (die Ausbeute beträft 72 Ma%). Die erhaltene Verbindung stellt eine weiße amorphe Substanz dar, die gut wasserlöslich, im Äthylalkohol begrenzt löslich ist. $R_f$ = 0,50 (das System B), die Haltezeit beträgt 5,1 min, das UV-Spektrum, nm, pH 7,0: $\lambda_{max}$ 273 ($\xi$8750), $\lambda_{min}$ 248 ($\xi$6200); 'H-NMR-Spektrum ($D_2O$,$\delta$,ppm): 7,62 d (IH,H6,$J_{5,6}$ = 8,0 Hz); 5,78 d (IH,H5,$J_{5,6}$ = 8,0 Hz); 5,95 t (IH,HI',$J_{I',2'}$ = 6,0 Hz); 2,70 m (2H,2H2'); 3,75-4,32 m (4H, H3' + H4' + 2H5'); 6,63 d (IH,$H^P$, $J_{P,H}$ = 632 Hz). $^{3I}$P-NMR ($D_2O$,$\delta$,ppm); 6,6 m ($J_{P,H}$ = 632 Hz, $J_{P,H}$5' = 6,3 Hz, $J_{P,H}$4' = 1,6 Hz).

Beispiel 7.

Ähnlich mit dem Beispiel 6 erhält man 3'-Azido-2',3'-didesoxyadenosin-5'-hydrophosphonat. Die Ausbeute beträgt 67 Ma%. Die erhaltene Verbindung stellt eine weiße amorphe Substanz dar, die gut wasserlöslich, im Äthylalkohol begrenzt löslich ist.

$R_f$ = 0,48 (das System B); die Haltezeit beträgt 8,9 min; das UV-Spektrum, nm, pH 7,0, $\lambda_{max}$ 260 (ξ15350) $\lambda_{min}$ 229 (ξ2700), 'H-NMR ($D_2O$,δ,ppm): 8,39 s (1H,H8); 8,25 s (IH,H2): 6,40 t (IH,HI', $J_{I,2}$ = 5,0 Hz)-;2,80m (2H,2H2');4,04-4,60m (4H,H3' + H4' + 2H5'); 6,65 d (IH,$H^P$, $J_{P,H}$ = 632 Hz). Das $^{31}$P-NMR-Spektrum ist dem Spektrum der erfindungsgemäßen Verbindung nach dem Beispiel 6 ähnlich.

Beispiel 8.

Ähnlich mit dem Beispiel 6 erhält man 3'-Azido-2',3'-didesoxyguanosin-5'-hydrophosphonat (die Ausbeute beträgt 58 Ma%). Die erhaltene Verbindung stellt eine weiße amorphe Substanz dar, die gut wasserlöslich, im Äthylalkohol begrenzt löslich ist.

$R_f$ = 0,44 (das System B); die Haltezeit beträgt 8,4 min, das UV-Spektrum, nm, pH 7,0: $\lambda_{max}$ 252 (ξII400), $\lambda_{min}$ 226 (ξ3000). 'H-NMR ($D_2O$,δ,ppm): 8,08 s (IH,H8); 6,42 t (IH,HI', $J_{I',2,}$ = 5,0 Hz; 2,80 m (2H,2H2'); 4,00-4,40 m (4H,H3' + H4' + 2H5'); 6,63 d (IH,$H^P$, $J_{P,H}$ = 654 Hz). $^{31}$P-NMR ($D_2O$,δ,ppm): 6,6 m ($J_{P,H}$ = 654 Hz, $J_{P,H}$5' = 6,4 Hz, $J_{P,H}$4' = 1,5 Hz).

Beispiel 9.

Zu einer Lösung von 3'-Azido-2',3'-didesoxythymidin (130 mg, 0,5 mMol) in 2 ml Trimethylphosphat gibt man bei einer Temperatur von 0 bis 4 °C innerhalb von 3 Stunden Dichlormethanophosphonat (200 mg, I,5 mMol) hinzu, rührt während der Nacht bei einer Temperatur von +4 °C und innerhalb von 5 Stunden bei einer Temperatur von 20 °C. Die Reaktionsmasse dampft man ein, gibt dem Rückstand unter Abkühlen auf 0 °C 5 ml Wasser und I ml Triäthylamin hinzu, läßt für I Stunde stehen und dampft wiederum ein. Man reinigt den Rückstand durch Chromatografie auf einer Kolonne (20x4 cm) mit dem Zellstoff DEAE in der $HCO_3^-$ -Form. Die Elution führt man mit 3 I linearem Ammoniumhydrogenkarbonatgradienten von 0 bis 0,1 M durch, dampft die Fraktionen ein, die das Endprodukt enthalten. Man entfernt den Salzüberschuß durch mehrmaliges Eindampfen mit Wasser. Man extrahiert den Rückstand mit 10 ml Methanol, konzentriert bis zum Erhalten eines Volumens von 2 ml, gibt 30 mg $NaClO_4$ und 5 ml Azeton hinzu. Den Rückstand trennt man ab, wäscht mit Azeton, trocknet. Man erhält 120 mg (33,0 Ma%) 3'-Azido-2',3'-didesoxythymidin-5'-methylphosphonat, das eine weiße amorphe Substanz darstellt die gut wasserlöslich, im Äthylalkohol begrenzt löslich ist. $R_f$ = 0,6 (das System B); die Haltezeit beträgt 7,0 min, das UV-Spektrum, nm, pH I,0: :$\lambda_{max}$ 265 (ξ9600), $\lambda_{min}$ 240 (ξ3450), 'H-NMR ($D_2O$, δ,ppm): 7,60 d (IH,H6, J = 0,5 Hz); I,88 d (3H,$CH_3$, J = 0,5 Hz); 6,16 t (IH,HI', $J_{I',2'}$ = 6,0 Hz); 2,46 m (2H, 2H2');

$J_{P,CH_3}$ =3,80-4,90 m (4H,H3'+H4'+2H5'); I,30 d (3H,$CH_3$P, $J_{P,CH_3}$ =I7 Hz).

Beispiel 10.

Zu einer Lösung von 3'-Azido-2',3'-didesoxy-N-benzoylcytidin (I87 mg, 0,5 mMol) in 2 ml Trimethylphosphat gibt man bei einer Temperatur von 0 bis 4 °C innerhalb von 3 Stunden Dichlormethanphosphonat (200 mg, 1,5 mMol) hinzu, rührt während der Nacht bei einer Temperatur von +4 °C und innerhalb von 5 Stunden bei einer Temperatur von 20 °C. Die Reaktionsmasse dampft man ein, gibt dem Rückstand unter Abkühlen auf 0 °C 5 ml Wasser und I ml Triäthylamin hinzu, läßt für I Stunde stehen und dampft ein. Zur Entfernung der N-Benzoylgruppe gibt man dem Rückstand 25 ml gesättigte methanolische Ammoniaklösung hinzu, dampft nach 24 Stunden die Lösung bis zur Trockne ein. Die Isolierung und Reinigung führt man nach der im Beispiel 9 beschriebenen Methodik durch.

Man erhält 3'-Azido-2',3'-didesoxycytidin-5'-methylphosphonat. Die Ausbeute beträgt 35 Ma%. Die erhaltene Verbindung stellt eine weiße amorphe Substanz, die gut wasserlöslich ist.

$R_f$ = 0,48 (das System B), die Haltezeit beträgt 4,6 min, das UV-Spektrum, nm, pH 7,0: $\lambda_{max}$ 27I (ξ8800), $\lambda_{min}$ 250 (ξ6400). 'H-NMR ($D_2O$,δ, ppm): 7,65 d (IH,H6,$J_{5,6}$ = = 8,0 Hz); 5,80 d (IH, H5, $J_{5,6}$ = 8,0 Hz); 5,97 t (IH,HI', $^{J}{}_{I',2,}$ = 6,0 Hz), 2,70 m (2H,2H2'), 3,78 - 4,34 m (H3' + H4' + 2H5'); 1,30 d (3H,$CH_3$P, $J_P$,$CH_3$ = I7 Hz).

Beispiel II.

Ähnlich mit dem Beispiel 10 erhält man 3'-Azido-2',3'-didesoxyadenosin-5'-methylphosphonat. Die Ausbeute beträgt 30 Ma%. Die erhaltene Verbindung stellt eine weiße amorphe, gut wasserlösliche Substanz dar.

$R_f = 0{,}38$ (das System B); die Haltezeit beträgt 7,8 min. Das UV-Spektrum, nm, pH 7,0: $\lambda_{max}$ 260 (ξI5300), $\lambda_{min}$ 230 (ξ2800), 'H-NMR ($D_2O$,δ,ppm): 8,24 s ((IH,H8); 6,40 t (IH,HI, $J_{I,2}$ =5,0 Hz); 2,80 m (2H,2H2'); 4,00-4,40 m (4H,H3' + H4' + 2H5');

$$I{,}30\ d\ (3H, CH_3P, J_{P,CH_3} = I7\ Hz).$$

Beispiel I2.

Ähnlich mit dem Beispiel I0 erhält man 3'-Azido-2',3'-didesoxguanosin-5'methylphosphonat. Die Ausbeute beträgt 30 Ma%. Die erhaltene Verbindung stellt eine weiße amorphe, gut wasserlösliche Substanz dar.

$R_f = 0{,}55$ (das System B); die Haltezeit beträgt 7,6 min. Das UV-Spektrum, nm, pH 7,0: $\lambda_{max}$ 253 (ξII000), $\lambda_{min}$ 228 (ξ2800).

'H-NMR ($D_2O$, δ,ppm): 8,08 s (IH,H8); 6,4 t (IH,HI, $J_{I,2}$ =5,0 Hz); 2,80 m (2H, 2H2'); 4,00-4,40 m (4H,H3' + H4' + 2H5');

$$I{,}30\ d\ (3H, CH_3P,\ J_{P,CH_3} = I{,}7\ Hz).$$

Industrielle Verwendbarkeit

Die erfindungsgemäßen Verbindungen 3'-Azido-2',3'-didesoxynukleosid-5'-phosphonate besitzen die Fähigkeit, die Reproduktion von Virus des menschlichen Immunodefizites in der Kultur der Lymphozyten zu hemmen, und können in der Medizin Verwendung finden.

## Patentansprüche

**1.** 3'-Azido-2',3'-didesoxynukleosid-5'-phosphonate der allgemeinen Formel:

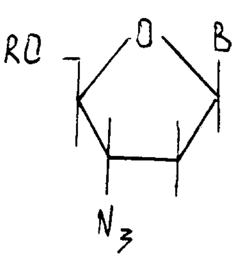

worin bedeuten:

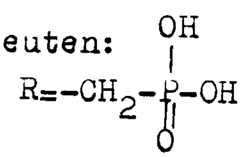

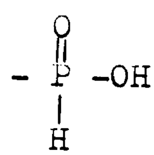

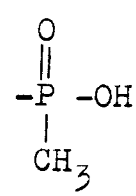

B = Thymin-I-yl, Cytosin-I-yl, Adenin-9-yl, Guanin-9-yl.

## Claims

**1.** 5'phosphonates of 3'-azido-2',3'-dideoxynucleosides of the general formula:

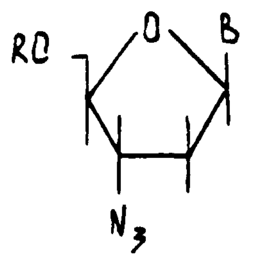

wherein:

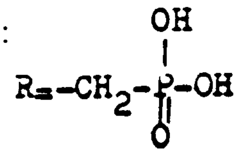

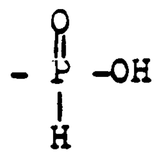

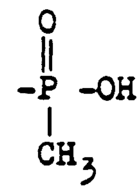

B = thymine-1-yl, cytosine-1-yl, adenine-9-yl, guanine-9-yl.

## Revendications

**1.** 3'-Azido-2',3'-didésoxynucléoside-5'-phosphonate de la formule générale :

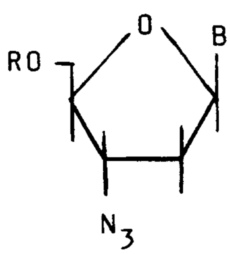

où R =

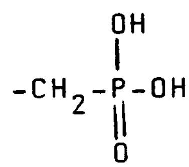

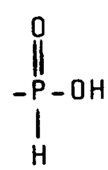

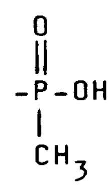

B = Thymin-1-yl, Cytosin-1-yl, Adénin-9-yl, Guanin-9-yl.